# EUROPEAN PATENT APPLICATION

(11) **EP 2 009 016 A2**
(43) Date of publication of application: **31.12.2008**
(21) Application number: 08013410.9
(22) Date of filing: 16.11.2007
(51) Int. Cl.: C07F 9/38

(54) **Process for preparing crystalline form of ibandronate sodium**

(30) Priority: 09.08.2007 US 954959 P; 27.12.2006 US 877572 P; 16.11.2006 US 859685 P; 06.11.2007 US 985837 P
(62) Divisional of application: 07853128.2
(71) Applicant: Teva Pharmaceutical Industries Ltd, 49131 Petah Tiqva (IL)
(72) Inventor: Turgeman, Eran, Herzella (IL); Lifshitz-Liron, Revital, 46739 Hertzlia (IL); Singer, Claude, 44358 Kfar Saba (IL); Koltai, Tamas, 42758 Netanya (IL)
(74) Representative: Russell, Tim

(57) **Abstract**

Provided is a process for the preparation of a crystalline form of ibandronate sodium.

## Description

### Cross-Reference to Related Application

This application claims the benefit of U.S. provisional application Serial Nos. 60/859,685, filed November 16, 2006; 60/877,572, filed December 27, 2006; 60/954,959, filed August 9, 2007; and 60/985837, filed November 6, 2007 each of which is hereby incorporated by reference in its entirety.

### Field of the Invention

The invention encompasses crystalline forms of ibandronate sodium, as well as processes for the preparation thereof.

### Background of the Invention

Ibandronate sodium, (1-hydroxy-3-(N-methyl-N-pentylamino)propylidene) bisphosphonic acid monosodium salt, is a third-generation nitrogen-containing bisphosphonate characterized by an aliphatic tertiary amine side chain. Ibandronate sodium is typically a white powder. Ibandronate sodium has the empirical formula C₉H₂₂NO₇P₂Na and the following chemical structure.

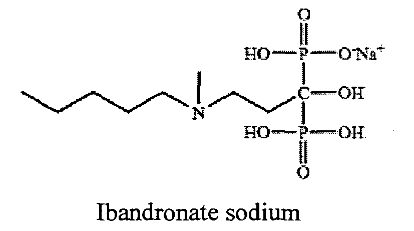

Ibandronate sodium is currently marketed in the United States by Hoffmann-La Roche under the tradename BONIVA^{®} in its monohydrate form. BONIVA^{®} is indicated for the treatment and prevention of osteoporosis in post-menopausal women. BONIVA^{®} is available as an intravenous injection administered every 2-3 months or as an oral formulation. BONIVA^{®} is marketed in Europe under the tradename BONDRONAT^{®} for the treatment of skeletal-related events in patients with breast cancer and bone metastases. BONDRONAT^{®} is available in an ampoule with 1 ml concentrate for solution for infusion; 1 ml of solution is reported to contain 1.125 mg of ibandronic monosodium salt monohydrate, corresponding to 1 mg of ibandronic acid.

Ibandronate salts, such as ibandronate sodium, are generally prepared from ibandronic acid ("IBD-Ac"), which has the following chemical structure:

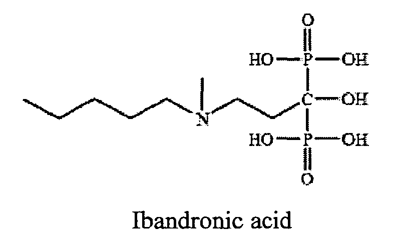

U.S. Patent No. 4,927,814 discloses diphosphonic acids, such as ibandronic acid, derivatives thereof, processes for preparing the acids and derivatives, and pharmaceutical compositions containing them.

The invention relates to the solid state physical properties of ibandronate sodium. These properties can be influenced by controlling the conditions under which ibandronate sodium is obtained in solid form. Solid state physical properties include, for example, the flowability of the milled solid. Flowability affects the ease with which the material is handled during processing into a pharmaceutical product. When particles of the powdered compound do not flow past each other easily, a formulation specialist must necessitate the use of glidants such as colloidal silicon dioxide, talc, starch, or tribasic calcium phosphate.

Another important solid state property of a pharmaceutical compound is its rate of dissolution in aqueous fluid. The rate of dissolution of an active ingredient in a patient's s stomach fluid can have therapeutic consequences since it imposes an upper limit on the rate at which an orally administered active ingredient can reach the patient's bloodstream. The rate of dissolution is also a consideration in formulation syrups, elixirs, and other liquid medicaments. The solid state form of a compound can also affect its behavior on compaction and its storage stability.

These practical physical characteristics are influenced by the conformation and orientation of molecules in the unit cell, which define a particular polymorphic form of a substance. The polymorphic form can give rise to thermal behavior different from that of the amorphous material or another polymorphic form. Thermal behavior is measured in the laboratory by such techniques as capillary melting point, thermogravimetric analysis ("TGA"), and differential scanning calorimetry ("DSC") and can be used to distinguish some polymorphic forms from others. A particular polymorphic form can also give rise to distinct spectroscopic properties that can be detectable by powder x-ray crystallography, solid state ¹³C NMR spectroscopy, and infrared spectrometry.

Generally, a crystalline solid has improved chemical and physical stability over the amorphous form, and forms with low crystallinity. Crystalline forms may also exhibit improved solubility, hygroscopicity, bulk properties, and/or flowability.

The discovery of new polymorphic forms of a pharmaceutically useful compound provides a new opportunity to improve the performance characteristics of a pharmaceutical product. It enlarges the repertoire of materials that a formulation scientist has available for designing, for example, a pharmaceutical dosage form of a drug with a targeted release profile or other desired characteristic.

PCT Publication No. WO 2006/024024 refers to several crystalline forms of ibandronate sodium and processes for their preparation.

There is a need in the art for additional polymorphic forms of ibandronate sodium.

### Summary of the Invention

In another embodiment, the invention encompasses a crystalline form of ibandronate sodium denominated Form V. Form V is characterized by x-ray powder diffraction reflections at 5.3, 17.2, 17.8 and 18.4 °2θ ± 0.2 °2θ. The crystalline ibandronate sodium Form V may be prepared by a process comprising storing ibandronate sodium Form C at about 20°C to about 30°C for more than about 3 months. Alternatively, the crystalline ibandronate sodium Form V may be prepared by a process comprising storing ibandronate sodium Form H at about 20°C to about 30°C, for more than about 3 months. The crystalline ibandronate sodium Form V may also be prepared by a process comprising: slurrying ibandronic acid and 1-propanol or 2-propanol; heating; adding and a base selected from the group consisting of: sodium tetraborate and sodium acetate; and cooling to obtain a precipitate.

In yet another embodiment, the invention encompasses a crystalline form of ibandronate sodium denominated Form W. Form W is characterized by x-ray powder diffraction reflections at 5.4, 11.4, 16.5 and 17.5 °2θ ± 0.2 °2θ. The crystalline ibandronate sodium Form W may be prepared by a process comprising: combining ibandronic acid, water, and a base selected from the group consisting of sodium hydroxide, Na₂CO₃, NaHCO₃, and NaOAc to obtain a solution; and combining the solution with isopropyl alcohol ("IPA") to obtain the crystalline ibandronate sodium Form W.

In one embodiment, the present invention encompasses a crystalline form of ibandronate sodium denominated Form L. Form L is characterized by x-ray powder diffraction reflections at 5.0, 10.9, 13.8 and 17.7 °2θ ± 0.2 °2θ. The crystalline ibandronate sodium Form L is prepared by a process comprising slurrying ibandronate sodium in 2-butanol.

In yet another embodiment, the invention encompasses a process for preparing crystalline ibandronate sodium Form G comprising combining ibandronate sodium and a solvent selected from the group consisting of dimethylsulfoxide ("DMSO") and ethanol to obtain a slurry of the crystalline ibandronate sodium Form G.

In one embodiment, the invention also encompasses a process for preparing crystalline ibandronate sodium Form Q comprising storing crystalline ibandronate sodium Form Q2, Form R, or Form S at a temperature of about 15°C to about 30°C for more than about 3 months.

In one embodiment, the invention encompasses a process for preparing crystalline ibandronate sodium Form QQ comprising storing crystalline ibandronate sodium crystalline Form F or Form L at about room temperature for more than about 3 months.

In another embodiment, the invention encompasses a process for preparing crystalline ibandronate sodium Form QQ comprising storing crystalline ibandronate sodium Form Q, Form Q3, Form Q4, Form K, or Form K3 at about room temperature for about one week at about 60-100 % RH.

In yet another embodiment, the invention encompasses a process for preparing crystalline ibandronate sodium Form T comprising storing amorphous ibandronate sodium at about room temperature for more than about 3 months.

In one embodiment, the invention encompasses a process for preparing crystalline ibandronate sodium Form Q4 comprising storing crystalline ibandronate sodium Form Q3 at about room temperature for more than about 3 months.

In yet another embodiment, the invention encompasses a process for preparing crystalline ibandronate sodium Form C comprising suspending ibandronic acid Form S1 in n-butanol or 2-butanol; heating the suspension; adding sodium tetraborate decahydrate to the suspension to obtain ibandronate sodium; and cooling the suspension to obtain a precipitate of the crystalline ibandronate sodium Form C.

In another embodiment, the invention encompasses crystalline ibandronate sodium Forms L, G, V, or W having a maximal particle size of less than about 500 µm, more preferably less than about 300 µm, even more preferably less than about 200 µm, even more preferably less than about 100 µm, and most preferably less than about 50 µm.

In yet another embodiment, the invention encompasses a pharmaceutical formulation comprising at least one of the above-described crystalline ibandronate sodium Forms V, W, or L and at least one pharmaceutically acceptable excipient.

In one embodiment, the invention encompasses a process for preparing a pharmaceutical formulation comprising combining at least one of the above-described crystalline ibandronate sodium Forms V, W, or L with at least one pharmaceutically acceptable excipient.

In one embodiment, the invention encompasses the use of the above-described crystalline ibandronate sodium Forms V, W, or L in the manufacture of a pharmaceutical composition.

In another embodiment, the invention encompasses at least one of the above-described crystalline ibandronate sodium Forms V, W, or L for use in treating or preventing skeletal-related events, preferably wherein the skeletal-related event is osteoporosis.

### Brief Description of the Drawings

Figure 1 illustrates a characteristic x-ray powder diffractogram of crystalline ibandronate sodium Form L.
Figure 2 illustrates a characteristic x-ray powder diffractogram of crystalline ibandronate sodium Form G.
Figure 3 illustrates a characteristic x-ray powder diffractogram of crystalline ibandronate sodium Form V.
Figure 4 illustrates a characteristic x-ray powder diffractogram of crystalline ibandronate sodium Form W.

### Detailed Description of the Invention

The invention addresses a need in the art by providing additional crystalline forms of ibandronate sodium, as well as processes for their preparation. The invention also provides additional processes for preparing known crystalline forms of ibandronate sodium.

As used herein, unless otherwise defined, the term "room temperature" refers to a temperature of about 15°C to about 30°C.

PCT Publication No. WO 2006/024024 ("WO '024"), hereby incorporated by reference, refers to the following crystalline forms of ibandronate sodium, as well as processes for preparing them: Form C, Form D, Form E, Form F, Form G, Form H, Form J, Form K, Form K2, Form K3, Form Q, Form Q1, Form Q2, Form Q3, Form Q4, Form Q5, Form Q6, Form QQ, Form R, Form S, Form T. WO '024 reports several characteristic powder x-ray diffraction ("PXRD") reflections for each crystalline form of ibandronate sodium. These characteristic reflections are summarized in Table 1 below.

**Table 1: Characteristic PXRD Reflections Reported in WO '024 for Crystalline Forms of Ibandronate Sodium**

| Crystalline Form | Primary Characteristic PXRD Reflections | Secondary Characteristic PXRD Reflections |
|---|---|---|
| Form C | 4.7, 5.0, 17.2, 18.3 and 19.5 °2θ± 0.2 °2θ | 17.6, 19.7, 20.2, 20.6, and 23.8 °2θ ± 0.2 °2θ |
| Form D | 4.8, 9.3, 18.5, 23.1, and 36.1 °2θ ± 0.2 °2θ | 15.3, 19.9, 26.3, 27.2, and 30.4 °2θ ± 0.2 °2θ |
| Form E | 4.6, 4.8, 5.3, 9.3, and 34.7 °2θ ± 0.2 °2θ | 18.6, 23.3, 24.5, 27.1, and 30.1 °2θ ± 0.2 °2θ |
| Form F | 4.9, 5.1, 6.0, 20.0, and 36.4 °2θ ± 0.2 °2θ | 18.6, 26.0, 28.5, 30.4, and 31.3 °2θ ± 0.2 °2θ |
| Form G | 4.7, 9.2, 17.4, 18.4, and 19.9 °2θ ± 0.2 °2θ | 10.1, 15.2, 18.7, 26.3, and 27.1 °2θ ± 0.2 °2θ |
| Form H | 4.8, 5.7, 17.3, 19.5, and 26.0 °2θ ± 0.2 °2θ | 18.5, 20.1, 23.8, 31.1, and 37.1 °2θ ± 0.2 °2θ |
| Form J | 4.6, 9.2, 18.3, 19.6, and 25.6 °2θ ± 0.2 °2θ | 17.5, 18.9, 21.7, 22.9, and 29.5 °2θ ± 0.2 °2θ |
| Form K | 5.0, 5.9, 17.2, 20.0, and 25.9 °2θ ± 0.2 °2θ | 18.5, 19.7, 21.4, 26.5, and 31.1 °2θ ± 0.2 °2θ |
| Form K2 | 5.1, 6.1, 17.3, 20.1, and 21.5 °2θ ± 0.2 °2θ | 18.6, 19.6, 26.1, 26.8, and 31.1 °2θ ± 0.2 °2θ |
| Form K3 | 5.1, 6.2, 17.3, 19.7, and 20.1 °2θ ± 0.2 °2θ | 18.5, 21.5, 23.8, 25.8, and 31.1 °2θ ± 0.2 °2θ |
| Form Q | 5.0, 6.1, 17.2, 25.7, and 30.9 °2θ ± 0.2 °2θ | 16.8, 21.4, 26.7, 29.1, and 36.9 °2θ ± 0.2 °2θ |
| Form Q1 | 4.7, 6.0, 17.2, 26.2, and 31.0 °2θ ± 0.2 °2θ | 19.5, 21.4, 25.8, 29.1, and 37.1 °2θ ± 0.2 °2θ |
| Form Q2 | 4.9, 6.2, 25.9, 31.0, and 37.1 °2θ ± 0.2 °2θ | 16.9, 17.3, 19.0, 26.6, and 29.2 °2θ ± 0.2 °2θ |
| Form Q3 | 5.9, 17.1, 19.6, 20.2, and 21.3 °2θ ± 0.2 °2θ | 18.0, 18.5, 23.6, 24.7, and 30.8 °2θ ± 0.2 °2θ |
| Form Q4 | 6.1, 17.2, 19.6, 20.3, and 21.4 °2θ ± 0.2 °2θ | 16.9, 18.1, 18.5, 23.7, and 24.8 °2θ ± 0.2 °2θ |
| Form Q5 | 6.1, 17.2, 19.6, 20.1, and 21.5 °2θ ± 0.2 °2θ | 16.8, 24.7, 25.7, 29.0, and 30.9 °2θ ± 0.2 °2θ |
| Form Q6 | 6.1, 17.3, 19.6, 21.5, and 30.8 °2θ ± 0.2 °2θ | 16.9, 20.2, 25.6, 26.9, and 29.1 °2θ ± 0.2 °2θ |
| Form QQ | 6.2, 25.9, 26.7, 31.1, and 37.2 °2θ ± 0.2 °2θ | 16.9, 17.3, 21.5, 24.7, and 29.2 °2θ ± 0.2 °2θ |
| Form R | 5.3, 6.0, 17.2, 18.7, and 20.0 °2θ ± 0.2 °2θ | 20.5, 25.0, 26.5, 29.1, and 31.0 °2θ ± 0.2 °2θ |
| Form S | 4.8, 5.1, 5.3, 5.4, and 6.1 °2θ ± 0.2 °2θ | 10.5, 21.0, 26.3, 33.0, and 38.2 °2θ ± 0.2 °2θ |
| Form T | 6.2, 15.7, 26.3, 32.6, and 35.6 °2θ ± 0.2 °2θ | 17.6, 19.4, 26.9, 31.7, and 38.7 °2θ ± 0.2 °2θ |

As used herein, Forms C, D, E, F, G, H, J, K, K2, K3, Q, Q1, Q2, Q3, Q4, Q5, Q6, QQ, R, S, and T of ibandronate sodium are as defined in WO2006024024, and can be made by the processes disclosed therein. Thus, as used herein ibandronic acid Forms C, D, E, F, G, H, J, K, K2, K3, Q, Q1, Q2, Q3, Q4, Q5, Q6, QQ, R, S, and T are characterized by the "primary" PXRD peaks as listed in the second column of Table 1 above, and may further be characterized by the "secondary" PXRD peaks listed in the third column of Table 1 above.

The invention encompasses additional crystalline forms of ibandronate sodium, denominated Forms V, W, and L.

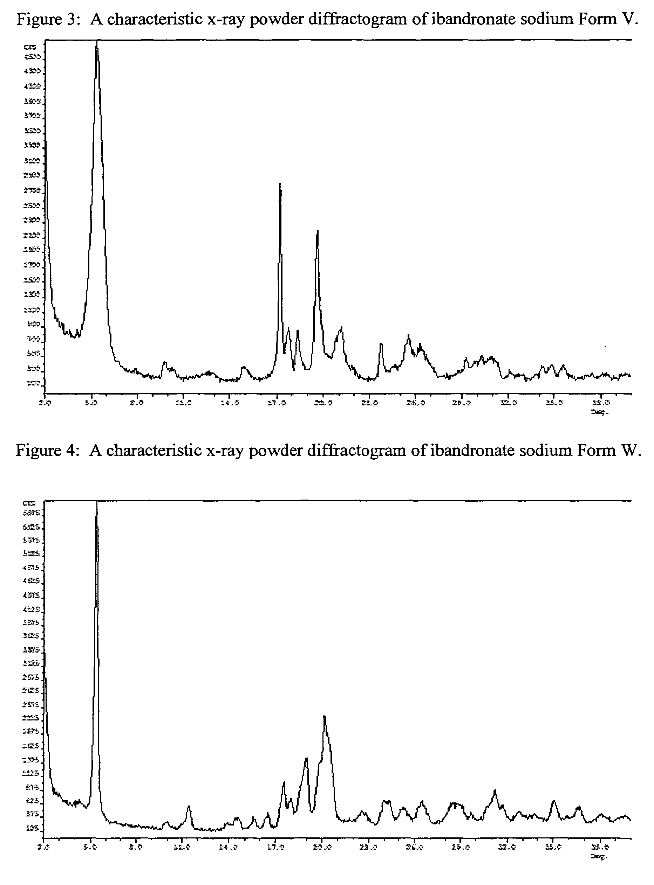

The invention encompasses a crystalline form of ibandronate sodium denominated Form V. Form V is characterized by x-ray powder diffraction reflections at 5.3, 17.2, 17.8 and 18.4 °2θ ± 0.2 °2θ. Form V can be further characterized by x-ray powder diffraction reflections at 19.6 and 21.2°2θ ± 0.2 °2θ. Figure 3 illustrates a representative powder x-ray diffraction diagram for Form V.

One of ordinary skill in the art is aware that there is a certain amount of experimental error inherent in PXRD techniques. *See, e.g.,* U.S. PHARMACOPOEIA, 387-89 (30th ed. 2007), hereby incorporated by reference. As to individual peaks, peak positions are reported over a range of ± 0.2° 20 to account for this experimental error. As to PXRD patterns in their entirety, the term "as depicted" in a particular figure is meant to account for this experimental error, as well as for variations in peak position and intensity due to factors such as, for example, variations in sample preparation, instrumentation, and the skill of the operator of the instrument. A PXRD pattern "as depicted" in a particular figure means that one of ordinary skill in the art, understanding the experimental error involved in powder X-ray diffraction techniques, would determine that the PXRD pattern corresponds to the same crystalline structure as the PXRD pattern depicted in the figure.

The crystalline ibandronate sodium Form V may be a solvate of 1-butanol, and may contain about 15 % of 1-butanol by weight as determined by Thermal Gravimetric Analysis (TGA). The crystalline bandronate sodium Form V may also be a solvate of 1 or 2-propanol and may contain about 16 % of 1 or 2-propanol, respectively, by weight as determined by TGA. The crystalline ibandronate sodium Form V may be a monobutanolate, monopropanolate or monoisopropanolate.

The crystalline ibandronate sodium Form V may be prepared by a process comprising storing crystalline ibandronate sodium Form C at about 20°C to about 30°C for more than about 3 months. Preferably, the temperature is about room temperature. Preferably, the ibandronate sodium Form C is stored at a relative humidity of about 40% to about 80%, and more preferably about 50% to about 70%.

Preferably, the crystalline ibandronate sodium Form C is stored for about 2 years.

Alternatively, the crystalline ibandronate sodium Form V may be prepared by a process comprising storing ibandronate sodium Form H at about 20°C to about 30°C, and preferably at about room temperature, for more than about 3 months. Preferably, the crystalline ibandronate sodium Form V is stored at a relative humidity of about 40% to about 80%, and preferably about 50% to about 70%. Preferably, the ibandronate sodium Form H is stored for about 2 years.

Alternatively, the crystalline ibandronate sodium Form V may be prepared by a process comprising slurrying ibandronic acid and 1-propanol or 2-propanol; heating the slurry; adding to the slurry a base selected from the group consisting of sodium tetraborate and sodium acetate; and cooling the slurry to obtain a precipitate of the crystalline ibandronate sodium Form V.

Prior to the heating step, the slurry is typically stirred.

Preferably, the slurry is heated to about 75°C to about 100°C, and more preferably to about reflux temperature. The slurry is typically stirred while heating. Preferably, the slurry is stirred while heating for about 1 hour to about 5 hours prior to the cooling step.

Preferably, the slurry is cooled to about 30°C to about 5°C, and more preferably to about room temperature. The slurry is typically stirred while cooling. Preferably, the slurry is stirred while cooling for about 10 hours to about 20 hours, and more preferably to about 16 hours.

The precipitated crystalline ibandronate sodium Form V may be recovered from the slurry by any method known to one of ordinary skill in the art. Preferably, the crystalline ibandronate sodium Form V is recovered by collecting the precipitate of crystalline ibandronate sodium Form V from the slurry by filtration, washing the precipitate, and drying the precipitate. Preferably, the precipitate is washed with the same solvent used in the process. Preferably, the precipitate is dried under vacuum with heating, preferably at a temperature of about 30°C to about 60°C, and more preferably at about 50°C. Preferably, the drying is done under a pressure of about 20 to about 30 mbar. The precipitate may be dried for about 19 hours to about 25 hours.

The invention also encompasses a crystalline form of ibandronate sodium denominated Form W. Form W is characterized by x-ray powder diffraction reflections at 5.4, 11.4, 16.5 and 17.5 °2θ ± 0.2 °2θ. Form W can be further characterized by x-ray powder diffraction reflections at 19.0 and 20.2 ± 0.2 °2θ ± 0.2 °2θ. Figure 4 illustrates a representative powder x-ray diffraction diagram for Form W.

The crystalline ibandronate sodium Form W may be prepared by a process comprising: combining ibandronic acid, water, and a base selected from the group consisting of sodium hydroxide, Na₂CO₃, NaHCO₃, and NaOAc to obtain a solution; and combining the solution with isopropyl alcohol ("IPA") to obtain the crystalline ibandronate sodium Form W.

Preferably, the combination of the ibandronic acid, water, and base is heated while stirring to obtain the solution. Preferably, the combination of the ibandronic acid, water, and base is heated at a temperature of about 70°C to about 80°C, and more preferably at a temperature of about 72°C.

Preferably, the solution is combined with the isopropyl alcohol by adding the solution to the isopropyl alcohol. Typically, the isopropyl alcohol is cooled prior to combining with the solution. Preferably, the isopropyl alcohol is cooled to a temperature of about 0°C to about -10°C, and more preferably to a temperature of about -2°C.

Typically, combining the solution with the isopropyl alcohol produces a mixture containing a precipitate of the crystalline ibandronate sodium Form W. Preferably, the mixture is stirred while cooling prior to recovering the crystalline ibandronate sodium Form W from the mixture. Preferably, the mixture is stirred for about 10 hours to about 24 hours, more preferably for about 15 hours to about 24 hours, and most preferably for about 17 hours. Preferably, the mixture is cooled to a temperature of about -5°C to about 5°C, and more preferably to a temperature of about 0°C.

The crystalline ibandronate sodium Form W may be recovered from the mixture by any method known to one of ordinary skill in the art. Preferably, the crystalline ibandronate sodium Form W is recovered by collecting the precipitate of crystalline ibandronate sodium Form W from the mixture by filtration, and drying the precipitate. Preferably, the precipitate is dried in a vacuum oven at a temperature of about 50°C to about 80°C, and more preferably at a temperature of about 50°C, for about 20 hours to about 30 hours, and more preferably for about 24 hours. Preferably, the drying is done under a pressure of about 10-200 mm Hg.

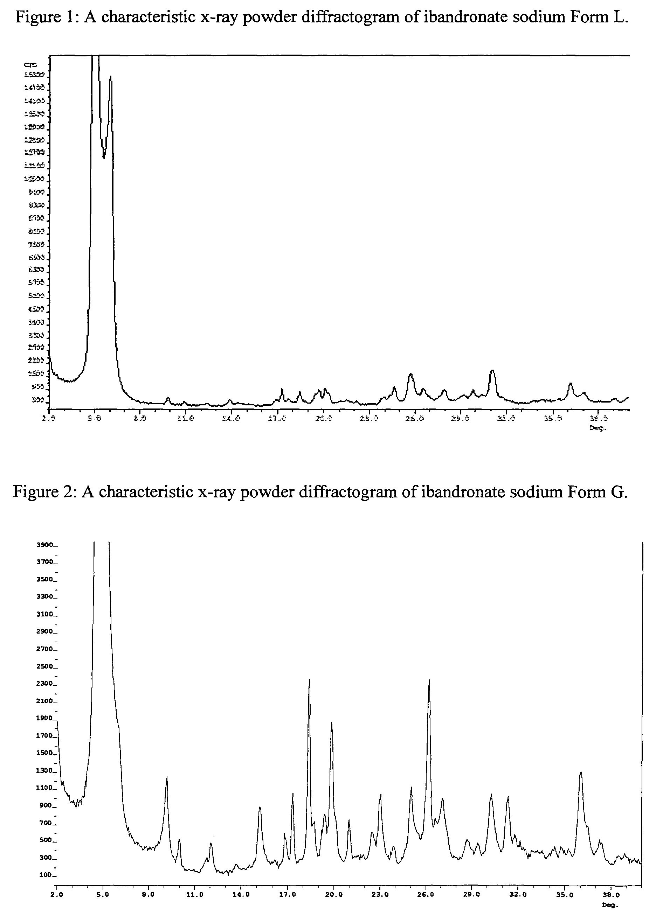

The invention also encompasses a crystalline form of ibandronate sodium denominated Form L. Form L is characterized by x-ray powder diffraction reflections at 5.0, 10.9, 13.8 and 17.7 °2θ ± 0.2 °2θ. Form L can be further characterized by an x-ray powder diffraction reflection at 27.9 °2θ ± 0.2 °2θ. Figure 1 illustrates a representative powder x-ray diffraction diagram for Form L.

The crystalline ibandronate sodium Form L is prepared by a process comprising slurrying crystalline ibandronate sodium Form QQ in 2-butanol.

Typically, the slurry of the ibandronate sodium in 2-butanol is maintained, with stirring, for a period of time sufficient to obtain the crystalline ibandronate sodium Form L. Preferably, the slurry is maintained, with stirring, for about 0 to about 24 hours, and more preferably for about 22 hours, to obtain the crystalline ibandronate sodium Form L. Preferably, the slurry is maintained at a temperature of about 100°C to about 110°C, and more preferably at about reflux temperature.

The crystalline ibandronate sodium Form L may be recovered from the slurry by any method known to one of ordinary skill in the art. Preferably, the crystalline ibandronate sodium Form L is recovered by collecting the precipitate from the slurry by filtration, washing the precipitate, and drying the precipitate. Preferably, the precipitate is washed with 2-butanol. Preferably, the precipitate is dried in a vacuum oven at a temperature of about 50°C to about 80°C, and more preferably at a temperature of about 50°C. Preferably, the drying is done under a pressure of about 10 mmHg to 200mmHg. Preferably, the drying is performed until a constant weight is obtained. More preferably, the drying is performed for about 10 hours to about 48 hours, and preferably for about 24 hours.

The invention also encompasses additional processes for preparing the crystalline ibandronate sodium Forms G, Q, QQ, T, Q4, and C referred to in WO '024.

The invention encompasses a process for preparing crystalline ibandronate sodium Form G comprising combining ibandronate sodium and a solvent selected from the group consisting of dimethylsulfoxide ("DMSO") and ethanol to obtain a slurry of the crystalline ibandronate sodium Form G.

Typically, the slurry is maintained, preferably with stirring, for a period of time sufficient to obtain the crystalline ibandronate sodium Form G. Preferably, the slurry is maintained with stirring for about 20 hours to about 30 hours, and more preferably for about 24 hours to about 26 hours, to obtain the crystalline ibandronate sodium Form G. Preferably, the slurry is maintained at about room temperature.

The crystalline ibandronate sodium Form G may be recovered by any method known to one of ordinary skill in the art. Preferably, the crystalline ibandronate sodium Form G is recovered by collecting the precipitate from the slurry by filtration, washing the precipitate, and drying the precipitate. Preferably, the precipitate is washed with the same solvent used to prepare the slurry. Preferably, the precipitate is dried under vacuum with heating, preferably at a temperature of about 50°C to about 80°C, and more preferably at a temperature of about 50°C. Preferably, the drying is done under a pressure of about 10-200 mm Hg.

The invention also encompasses a process for preparing crystalline ibandronate sodium Form Q comprising storing crystalline ibandronate sodium Form Q2, Form R, or Form S at a temperature of about 15°C to about 30°C for more than about 3 months. Preferably, the ibandronate sodium is stored at about room temperature. Preferably, the ibandronate sodium is stored for about 2 years. The crystalline ibandronate sodium Form Q2, Form R, or Form S may be prepared by the processes provided in WO '024.

The invention also encompasses a process for preparing crystalline ibandronate sodium Form QQ comprising storing crystalline ibandronate sodium crystalline Form F or Form L at about room temperature for more than about 3 months. Preferably, the ibandronate sodium is stored at about room temperature. Preferably, the ibandronate sodium is stored for about 2 years. The crystalline ibandronate sodium Form F may be prepared by the process provided in WO '024.

The invention also encompasses a process for preparing crystalline ibandronate sodium Form QQ comprising storing crystalline ibandronate sodium Form Q, Form Q3, Form Q4, Form K, or Form K3 at about room temperature for about one week at about 60-100 % relative humidity. The crystalline ibandronate sodium Form Q, Form Q3, Form Q4, Form K, or Form K3 may be prepared by the processes provided in WO '024.

The invention also encompasses a process for preparing crystalline ibandronate sodium Form T comprising storing amorphous ibandronate sodium at about room temperature for more than about 3 months. Preferably, the ibandronate sodium is stored for about 2 years. The amorphous ibandronate sodium may be prepared by the process provided in WO '024.

The invention also encompasses a process for preparing crystalline ibandronate sodium Form Q4 comprising storing crystalline ibandronate sodium Form Q3 at about room temperature for more than about 3 months. Preferably, the ibandronate sodium is stored for about 2 years. The crystalline ibandronate sodium Form Q3 may be prepared by the process provided in WO '024.

The invention also encompasses a process for preparing crystalline ibandronate sodium Form C comprising suspending ibandronic acid Form S1 in n-butanol or 2-butanol; heating the suspension; adding sodium tetraborate decahydrate to the suspension to obtain ibandronate sodium; and cooling the suspension to obtain a precipitate of the crystalline ibandronate sodium Form C.

The ibandronic acid Form S1 may be prepared by the process disclosed in PCT Publication No. WO 2006/002348, hereby incorporated by reference. Ibandronic acid Form S1 is typically characterized by x-ray powder diffraction reflections at 8.2, 11.5, 11.9, 13.9, 18.6 and 22.2 °2θ± 0.2 °2θ.

Prior to the heating step, the suspension is typically stirred. Preferably, the suspension is heated to a temperature of about 85°C to about 115°C, and more preferably to a temperature of about 114°C The suspension is typically stirred while heating. Preferably, the suspension is stirred while heating for about 1 hour to about 5 hours prior to the cooling step.

Preferably, the suspension is cooled to a temperature of about 30°C to about 10°C, and more preferably to about room temperature. The suspension is typically stirred while cooling. Preferably, the suspension is stirred while cooling for about 10 hours to about 24 hours, and more preferably for about 16 hours.

The precipitated crystalline ibandronate sodium Form C may be recovered from the suspension by any method known to one of ordinary skill in the art. Preferably, the crystalline ibandronate sodium Form C is recovered by collecting the precipitate of crystalline ibandronate sodium Form C from the suspension by filtration, washing the precipitate, and drying the precipitate. Preferably, the precipitate is washed with n-butanol. Preferably, the precipitate is dried under vacuum with heating, preferably at a temperature of about 85°C to about 115°C, and more preferably at a temperature of about 50°C. The precipitate may be dried for about 19 hours to about 20 hours. Preferably, the drying is done under a pressure of about 10-200 mm Hg.

Crystalline ibandronate sodium Forms D, J, E, QQ, K3, Q4, G, K and Q6 were found to be stable at a temperature of about 15°C to about 30°C for more than about 3 months, as described in Example 7 below.

The invention also encompasses crystalline ibandronate sodium Forms L, G, V, or W having a maximal particle size of less than about 500 µm, more preferably less than about 300 11m, even more preferably less than about 200 µm, even more preferably less than about 100 µm, and most preferably less than about 50 µm. As used herein, unless otherwise defined, the term "maximal particle size," when used to described a sample of crystalline ibandronate sodium, means that 99% of the particles in the sample have a particle size of less than or equal to the maximal particle size. The particle size of the ibandronate sodium crystalline forms may be measured by methods such as sieves, sedimentation, electrozone sensing (coulter counter), microscopy, and/or Low Angle Laser Light Scattering (LALLS).

The invention also encompasses a pharmaceutical formulation comprising at least one of the above-described crystalline ibandronate sodium Forms V, W, or L, and at least one pharmaceutically acceptable excipient.

The invention further encompasses a process for preparing a pharmaceutical formulation comprising combining at least one of the above-described crystalline ibandronate sodium Forms V, W, or L with at least one pharmaceutically acceptable excipient.

The invention further encompasses the use of the above-described crystalline ibandronate sodium Forms V, W, or L in the manufacture of a pharmaceutical composition.

Pharmaceutical formulations of the invention contain crystalline ibandronate sodium, such as one of the above-described forms, and optionally one or more other forms of ibandronate sodium. In addition to the active ingredient, the pharmaceutical formulations of the invention can contain one or more excipients. Excipients are added to the formulation for a variety of purposes.

Diluents increase the bulk of a solid pharmaceutical composition, and can make a pharmaceutical dosage form containing the composition easier for the patient and caregiver to handle. Diluents for solid compositions include, for example, microcrystalline cellulose (e.g. AVICEL^{®}), microfine cellulose, lactose, starch, pregelatinized starch, calcium carbonate, calcium sulfate, sugar, dextrates, dextrin, dextrose, dibasic calcium phosphate dihydrate, tribasic calcium phosphate, kaolin, magnesium carbonate, magnesium oxide, maltodextrin, mannitol, polymethacrylates (e.g. EUDRAGIT^{®}), potassium chloride, powdered cellulose, sodium chloride, sorbitol, and talc.

Solid pharmaceutical compositions that are compacted into a dosage form, such as a tablet, can include excipients whose functions include helping to bind the active ingredient and other excipients together after compression. Binders for solid pharmaceutical compositions include acacia, alginic acid, carbomer (e.g. CARBOPOL^{®}), carboxymethylcellulose sodium, dextrin, ethyl cellulose, gelatin, guar gum, hydrogenated vegetable oil, hydroxyethyl cellulose, hydroxypropyl cellulose (e.g. KLUCEL^{®}), hydroxypropyl methyl cellulose (e.g. METHOCEL^{®}), liquid glucose, magnesium aluminum silicate, maltodextrin, methylcellulose, polymethacrylates, povidone (e.g. KOLLIDON^{®}, PLASDONE^{®}), pregelatinized starch, sodium alginate, and starch.

The dissolution rate of a compacted solid pharmaceutical composition in the patient's stomach can be increased by the addition of a disintegrant to the composition. Disintegrants include alginic acid, carboxymethylcellulose calcium, carboxymethylcellulose sodium (e.g. AC-DT-SOL^{®}, PRIMELLOSE^{®}), colloidal silicon dioxide, croscarmellose sodium, crospovidone (e.g. KOLLIDON^{®}, POLYPLASDONE^{®}), guar gum, magnesium aluminum silicate, methyl cellulose, microcrystalline cellulose, polacrilin potassium, powdered cellulose, pregelatinized starch, sodium alginate, sodium starch glycolate (e.g. EXPLOTAB^{®}), and starch.

Glidants can be added to improve the flowability of a non-compacted solid composition and to improve the accuracy of dosing. Excipients that can function as glidants include colloidal silicon dioxide, magnesium trisilicate, powdered cellulose, starch, talc, and tribasic calcium phosphate.

When a dosage form such as a tablet is made by the compaction of a powdered composition, the composition is subjected to pressure from a punch and dye. Some excipients and active ingredients have a tendency to adhere to the surfaces of the punch and dye, which can cause the product to have pitting and other surface irregularities. A lubricant can be added to the composition to reduce adhesion and ease the release of the product from the dye. Lubricants include magnesium stearate, calcium stearate, glyceryl monostearate, glyceryl palmitostearate, hydrogenated castor oil, hydrogenated vegetable oil, mineral oil, polyethylene glycol, sodium benzoate, sodium lauryl sulfate, sodium stearyl fumarate, stearic acid, talc, and zinc stearate.

Flavoring agents and flavor enhancers make the dosage form more palatable to the patient. Common flavoring agents and flavor enhancers for pharmaceutical products that can be included in the composition of the invention include maltol, vanillin, ethyl vanillin, menthol, citric acid, fumaric acid, ethyl maltol, and tartaric acid.

Solid compositions can also be dyed using any pharmaceutically acceptable colorant to improve their appearance and/or facilitate patient identification of the product and unit dosage level.

The solid compositions of the invention include powders, granulates, aggregates, and compacted compositions. The dosages include dosages suitable for oral, buccal, rectal, parenteral (including subcutaneous, intramuscular, and intravenous), inhalant, and ophthalinic administration. The dosages can be conveniently presented in unit dosage form and prepared by any of the methods well-known in the pharmaceutical arts.

Solid dosage forms include tablets, powders, capsules, suppositories, sachets, troches, and lozenges, as well as suspensions.

The dosage form of the invention can be a capsule containing the composition, preferably a powdered or granulated solid composition of the invention, within either a hard or soft shell. The shell can be made from gelatin and optionally contain a plasticizer such as glycerin and sorbitol, and an opacifying agent or colorant.

The active ingredient and excipients can be formulated into compositions and dosage forms according to methods known in the art.

A composition for tableting or capsule filling can be prepared by wet granulation. In wet granulation, some or all of the active ingredients and excipients in powder form are blended and then further mixed in the presence of a liquid, typically water, that causes the powders to clump into granules. The granulate is screened and/or milled, dried, and then screened and/or milled to the desired particle size. The granulate can then be tableted, or other excipients can be added prior to tableting, such as a glidant and/or a lubricant.

A tableting composition can be prepared conventionally by dry blending. For example, the blended composition of the actives and excipients can be compacted into a slug or a sheet and then comminuted into compacted granules. The compacted granules can subsequently be compressed into a tablet.

As an alternative to dry granulation, a blended composition can be compressed directly into a compacted dosage form using direct compression techniques. Direct compression produces a more uniform tablet without granules. Excipients that are particularly well suited for direct compression tableting include microcrystalline cellulose, spray dried lactose, dicalcium phosphate dihydrate, and colloidal silica. The proper use of these and other excipients in direct compression tableting is known to those in the art with experience and skill in particular formulation challenges of direct compression tableting.

A capsule filling of the invention can comprise any of the aforementioned blends and granulates that were described with reference to tableting, but they are not subjected to a final tableting step.

The invention also encompasses methods of treating or preventing skeletal-related events, such as osteoporosis, comprising administering a pharmaceutical formulation comprising a therapeutically effective amount of at least one of the above-described crystalline forms of ibandronate sodium and at least one pharmaceutically acceptable excipient to a patient in need thereof. Ibandronate sodium may be formulated for administration to a mammal, preferably a human, by injection. The crystalline ibandronate sodium can be formulated, for example, as a suspension for injection. The formulation can contain one or more solvents. A suitable solvent can be selected by considering the solvent's physical and chemical stability at various pH levels, viscosity (which would allow for syringeability), fluidity, boiling point, miscibility, and purity. Suitable solvents include alcohol USP, benzyl alcohol NF, benzyl benzoate USP, and Castor oil USP. Additional substances can be added to the formulation such as buffers, solubilizers, and antioxidants, among others. *See, e.g.,* Ansel, H.C., et al., Pharmaceutical Dosage Forms and Drug Delivery Systems (7th ed. 1999), which is incorporated herein by reference.

BONIVA^{®} and/or BONDRONAT^{®} can be used as guidance for formulation. BONIVA^{®} is available as an intravenous injection administered every 2-3 months and as an oral formulation. BONDRONAT^{®} is available in ampoule with 1 ml concentrate for solution for infusion. 1 ml of the solution contains 1.125 mg of ibandronic monosodium salt monohydrate, corresponding to 1 mg of ibandronic acid.

Having thus described the invention with reference to particular preferred embodiments, those in the art can appreciate modifications to the invention as described and illustrated that do not depart from the scope of the invention as disclosed in the specification. The following examples are set forth to aid in understanding the invention but are not intended to, and should not be construed to, limit its scope in any way. The examples do not include detailed descriptions of conventional methods. Such methods are well known to those of ordinary skill in the art and are described in numerous publications. Brittain, H.G., Polymorphism in Pharmaceutical Solids, Drugs and the Pharmaceutical Sciences, vol. 95 (Marcel Dekker, Inc. 1999) can be used for guidance. All references mentioned herein are incorporated in their entirety.

### Examples

### X-ray powder diffraction:

The x-ray powder diffraction was performed on Scintag X-ray powder diffractometer model X'TRA with a solid state detector at room temperature. Copper radiation of 1.5418 Å was used. The sample holder was a round standard aluminum sample holder with rough zero background. The scanning parameters were range: 2-40 degrees two-theta; scan mode: continuous scan; step size: 0.05 degrees; and at a rate of 5 degrees/minute.

### Thermal Gravimetric Analysts:

Typically to determine the Loss on Drying (LOD) by Thermal Gravimetric Analysis (TGA), a sample was heated from about 25°C to about 250°C at a heating rate of about 10°C per minute, while purging with nitrogen gas at a flow rate of 40 ml/min.

### Example 1: Process for preparing crystalline ibandronate sodium Form L

Ibandronate Sodium (3.0 g) was stirred in 2-Butanol (32 ml) at reflux temperature for 22 hours. The slurry was cooled to room temperature. The white solid was isolated by vacuum filtration, washed with 2-Butanol (2x25 ml) and dried in a vacuum oven at 50°C for 20 hours to obtain 3.0 g of crystalline ibandronate sodium form L.

### Example 2: Process for preparing crystalline ibandronate sodium Form G

Ibandronate Sodium (3.0 g) was stirred in DMSO (20 ml) at room temperature for 26 hours. The gelatinous product was then isolated by vacuum filtration, washed with DMSO (2 ml) and dried in a vacuum oven at 50°C for 24 hours to obtain 3.3 g of crystalline ibandronate sodium Form G.

### Example 3: Process for preparing crystalline ibandronate sodium Form G

Ibandronate Sodium (3.0 g) was stirred in Ethanol (20 ml) at room temperature for 25 hours. The white solid was then isolated by vacuum filtration, washed with Ethanol (2x5 ml) and dried in a vacuum oven at 50°C for 24 hours to obtain 3.4 g of crystalline ibandronate sodium Form G.

### Example 4: Process for preparing crystalline ibandronate sodium Form G

Ibandronate Sodium (3.0 g) was stirred in DMSO (5 ml) at room temperature for 25 hours. The gelatinous product was then isolated by vacuum filtration, washed with DMSO (6 ml) and dried in a vacuum oven at 50°C for 24 hours to obtain 3.2 g of crystalline ibandronate sodium Form G.

### Example 5: Process for preparing crystalline ibandronate sodium Form V

Crystalline ibandronate sodium Form C was stored at room temperature for 2 years. The sample was retested after the storage time and found to be crystalline ibandronate sodium Form V by XRD.

### Example 6: Process for preparing Ibandronate sodium Form V

Crystalline ibandronate Sodium Form H was stored at room temperature for 2 years. The sample was retested after the storage time and found to be a mixture of crystalline ibandronate sodium Forms H and V by XRD.

### Example 7: Stability of crystalline forms of ibandronate sodium when stored for 2 years at room temperature

1 g samples of crystalline ibandronate sodium Forms K1, D, J, H, E, Q2, QQ, K3, Q4, L. G. C, S, R, Q3, K, and Q6, as well as amorphous form were each placed into separate glass bottles, which were then closed and stored at room temperature for 2 years.

Table 2 summarizes the results before and after the storage.

**Table 2: Storage Stability at Room Temperature for Two Years**

| Crystal form at time 0 | Crystal form after 2 years |
|---|---|
| QQ | QQ |
| K1 | K |
| D | D |
| J | J |
| H | H+V |
| E | E |
| Q2 | Q |
| QQ | QQ |
| K3 | K3 |
| Q4 | Q4 |
| L | QQ |
| G | G |
| C | V |
| S | Q plus additional XRD reflections at 10.4, 11.3, 26.3, 27.9, and 31.6 °2θ ± 0.2 °2θ |
| R | Q |
| Amorphous | T |
| Q3 | Q4 |
| K | K |
| Q6 | Q6 |

As illustrated by Table 2 above, Forms D, J, E, QQ, K3, Q4, G, K, and Q6 maintained their crystalline form after storage at room temperature for two years.

### Example 8: Stability of crystalline forms of ibandronate sodium when stored at 0-100 % relative humidity

0.5 g samples of crystalline ibandronate sodium Forms Q, Q3, Q4, K3, and K were each placed into separate containers, which were then and stored at room temperature and 0%, 80%, and 100% relative humidity for one week.

Table 3 summarizes the results before and after the storage.

**Table 3: Storage Stability at Room Temperature and 0-100% Relative Humidity**

| Crystal form at time 0 | RH [%] | Crystal form after storage |
|---|---|---|
| Q | 80 | QQ |
| | 100 | QQ |
| Q3 | 80 | QQ |
| | 100 | QQ |
| Q4 | 100 | QQ |
| K3 | 0 | K3 |
| | 100 | QQ |
| K | 0 | K3 |
| | 100 | QQ |

### Example 9: Process for preparing crystalline ibandronate sodium Form W

A solution of Ibandronic acid (40 g) in water (370 ml) and Sodium hydroxide (5.05 g, solid) was stirred at 72°C. The solution was poured into cold IPA [7400 ml; T jacket = (-2°C)]. The obtained precipitate was stirred at 0°C for 17 hours. The precipitate was isolated by vacuum filtration, and dried in a vacuum oven at 50°C for 24 hours to obtain 32.7 g of crystalline ibandronate sodium Form W.

### Example 10: Process for preparing crystalline ibandronate sodium Form C

Ibandronic Acid Form S1 (5.0g) was stirred in n-butanol (100ml) at 114°C. To the obtained partial solution, sodium tetraborate decahydrate (3 g, 1eq) was added. The obtained slurry was stirred at 114°C for 3 hours and then it was cooled to room temperature. The slurry was stirred at room temperature for 16 hours. The precipitate was isolated by vacuum filtration, washed with n-butanol (2x5ml) and dried in a vacuum oven at 50°C for 19.5 hours to obtain 6.2g of crystalline ibandronate sodium Form C.

### Example 11: Process for preparing crystalline Ibandronate sodium Form V

Ibandronic Acid (5.0g) was stirred in 1-Propanol (100ml) at reflux temperature. Sodium tetraborate (1.6g, 1/2eq) (prepared according to examples 11(a) and 11(b)) was added. The slurry was stirred for 4 hours at reflux temperature. Then the slurry was cooled to room temperature. The slurry was stirred at room temperature for 16 hours. The precipitate was isolated by vacuum filtration, washed with 1-Propanol (2x5ml) and dried in a vacuum oven at 50°C for 24 hours to obtain 6g of crystalline Ibandronate Sodium Form V.

### Example 11(a): Process for preparing sodium tetraborate anhydrous

Sodium tetraborate decahydrate (Aldrich) (20g) and Toluene (Tech) (100ml) were stirred with mechanical stirrer and heated to reflux for azeotropic distillation with a dean stark apparatus for 3 hours. The slurry was cooled to room temperature and evaporated until dryness to give 13.45g sodium tetraborate anhydrous.

### Example 11(b): Process for preparing sodium tetraborate anhydrous

Sodium tetraborate decahydrate (Aldrich) (5g) and Toluene (Tech) (50ml) were stirred with a mechanical stirrer and heated to reflux for azeotropic distillation with a dean stark apparatus for 4 hours. The slurry was cooled to room temperature and stirred at room temperature over the weekend. The slurry was then evaporated until dryness to give 3g sodium tetraborate anhydrous.

### Example 12: Process for preparing crystalline Ibandronate sodium Form V

Ibandronic Acid (5.0g) was stirred in 2-Propanol (100ml) at reflux temperature. Sodium tetraborate (1.6g, 1/2eq) (prepared according to example 12(a)) was added. The slurry was stirred for 4 hours at reflux temperature. Then the slurry was cooled to room temperature. The slurry was stirred at room temperature for 16 hours. The precipitate was isolated by vacuum filtration, washed with 2-Propanol (2x10ml) and dried in a vacuum oven at 50°C for 24 hours to obtain 2.15g of crystalline Ibandronate Sodium form V.

### Example 12(a): Process for preparing sodium tetraborate anhydrous

Sodium tetraborate decahydrate (Aldrich) (20g) and Toluene (Tech) (100ml) were stirred with mechanical stirrer and heated to reflux for azeotropic distillation with a dean stark apparatus for 3 hours. The slurry was cooled to room temperature and evaporated until dryness to give 13.45g sodium tetraborate anhydrous.

### Example 13: Process for preparing Ibandronate sodium Form V

Ibandronic Acid (5.0g) was stirred in 2-Propanol (100ml) at reflux temperature. Sodium acetate (1.3g, 1eq) (prepared according to example 13(a)) was added. The slurry was stirred for 4 hours at reflux temperature. Then the slurry was cooled to room temperature. The slurry was stirred at room temperature for 16 hours. The precipitate was isolated by vacuum filtration, washed with 2-Propanol (2x5ml) and dried in a vacuum oven at 50°C for 24 hours to obtain 6g of crystalline Ibandronate Sodium Form V.

### Example 13(a): Process for preparing sodium acetate anhydrous

Sodium acetate (10g) and Toluene (Tech) (50ml) were stirred with a mechanical stirrer and heated to reflux for azeotropic distillation with a dean stark apparatus for 2.5 hours. The slurry was cooled to room temperature and evaporated until dryness to give 7.5g sodium acetate anhydrous.

Further aspects and features of the present invention are set out in the following numbered clauses.
1. A crystalline form of ibandronate sodium characterized by x-ray powder diffraction reflections at 5.3, 17.2, 17.8 and 18.4 °2θ ± 0.2 °2θ.
2. The crystalline form of ibandronate sodium of clause 1, further characterized by x-ray powder diffraction reflections at 19.6 and 21.2°2θ ± 0.2 °2θ.
3. The crystalline form of ibandronate sodium of clause 1 or 2, further characterized by a powder x-ray diffraction pattern as depicted in Figure 3.
4. The crystalline form of ibandronate sodium of any one of clauses 1 to 3, having a maximal particle size of less than about 500 µm.
5. The crystalline form of ibandronate sodium of any one of clauses 1 to 4, in the form of a solvate with 1-butanol, 2-propanol, or 1-propanol.
6. The crystalline form of ibandronate sodium of clause 5, wherein the solvate is a monosolvate.
7. A process for preparing a crystalline form of ibandronate sodium characterized by x-ray powder diffraction reflections at 5.3, 17.2, 17.8 and 18.4 °2θ ± 0.2 °2θ comprising storing a crystalline form of ibandronate sodium characterized by x-ray powder diffraction reflections at 4.7, 5.0, 17.2, 18.3 and 19.5 °2θ ± 0.2 °2θ at about 20°C to about 30°C for more than about 3 months.
8. The process of clause 7, wherein the crystalline form of ibandronate sodium characterized by x-ray powder diffraction reflections at 4.7, 5.0, 17.2, 18.3 and 19.5 °2θ ± 0.2 °2θ is stored for about 2 years.
9. The process of clause 7 or 8, wherein the crystalline form of ibandronate sodium characterized by x-ray powder diffraction reflections at 4.7, 5.0, 17.2, 18.3 and 19.5 °2θ ± 0.2 °2θ is stored at a relative humidity of about 40% to about 80%.
10. A process for preparing a crystalline form of ibandronate sodium characterized by x-ray powder diffraction reflections at 5.3, 17.2, 17.8 and 18.4 °2θ ± 0.2 °2θ comprising storing a crystalline form of ibandronate sodium characterized by x-ray powder diffraction reflections at 4.8, 5.7, 17.3, 19.5, and 26.0 °2θ ± 0.2 °2θ at about 20°C to about 30°C for more than about 3 months.
11. The process of clause 10, wherein the crystalline form of ibandronate sodium characterized by x-ray powder diffraction reflections at 4.8, 5.7, 17.3, 19.5, and 26.0 °2θ ± 0.2 °2θ is stored for about 2 years.
12. The process of clause 10 or 11, wherein the crystalline form of ibandronate sodium characterized by x-ray powder diffraction reflections at 4.8, 5.7, 17.3, 19.5, and 26.0 °2θ ± 0.2 °2θ is stored at a relative humidity of about 40% to about 80%.
13. A process for preparing a crystalline form of ibandronate sodium characterized by x-ray powder diffraction reflections at 5.3, 17.2, 17.8 and 18.4 °2θ ± 0.2 °2θ comprising slurrying ibandronic acid and 1-propanol or 2-propanol; heating the slurry; adding to the slurry a base selected from the group consisting of sodium tetraborate and sodium acetate; and cooling the slurry to obtain a precipitate of the crystalline ibandronate sodium.
14. The process of clause 13, wherein the slurry is heated to about reflux temperature.
15. The process of clause 13 or 14, wherein the slurry is cooled to a temperature of about 30°C to about 5°C.
16. A crystalline form of ibandronate sodium characterized by x-ray powder diffraction reflections at 5.4, 11.4, 16.5 and 17.5 °2θ ± 0.2 °2θ.
17. The crystalline form of ibandronate sodium of clause 16, further characterized by x-ray powder diffraction reflections at 19.0 and 20.2 ± 0.2 °2θ ± 0.2 °2θ.
18. The crystalline form of ibandronate sodium of clause 16 or 17, further characterized by a powder x-ray diffraction pattern as depicted in Figure 4.
19. The crystalline form of ibandronate sodium of any one of clauses 16 to 18, having a maximal particle size of less than about 500 µm.
20. A process for preparing a crystalline form of ibandronate sodium characterized by x-ray powder diffraction reflections at 5.4, 11.4, 16.5 and 17.5 °2θ ± 0.2 °2θ comprising combining ibandronic acid, water, and a base to obtain a solution; and combining the solution with isopropyl alcohol to obtain the crystalline form of ibandronate sodium,
   wherein the base is selected from the group consisting of sodium hydroxide, sodium carbonate, sodium bicarbonate, and sodium acetate.
21. The process of clause 20, wherein the combination of the ibandronic acid, water, and base is heated while stirring to obtain the solution.
22. The process of clause 21, wherein the combination of the ibandronic acid, water, and base is heated at a temperature of about 70°C to about 80°C.
23. The process of clause 21 or 22, wherein the isopropyl alcohol is cooled prior to combining with the solution.
24. The process of clause 23, wherein the isopropyl alcohol is cooled to a temperature of about 0°C to about -10°C.
25. The process of any one of clauses 20 to 24, further comprising cooling the combination of the solution and the isopropyl alcohol to precipitate the crystalline form of ibandronate sodium.
26. The process of clause 25, wherein the combination of the solution and the isopropyl alcohol is cooled to a temperature of about -5°C to about 5°C.
27. A crystalline form of ibandronate sodium characterized by x-ray powder diffraction reflections at 5.0, 10.9, 13.8 and 17.7 °2θ ± 0.2 °2θ.
28. The crystalline form of ibandronate sodium of clause 27, further characterized by an x-ray powder diffraction reflection at 27.9 °2θ ± 0.2 °2θ.
29. The crystalline form of ibandronate sodium of clause 27 or 28, further characterized by a powder x-ray diffraction pattern as depicted in Figure 1.
30. The crystalline form of ibandronate sodium of any one of clauses 27 to 29, having a maximal particle size of less than about 500 µm.
31. A process for preparing a crystalline form of ibandronate sodium characterized by x-ray powder diffraction reflections at 5.0, 10.9, 13.8 and 17.7 °2θ ± 0.2 °2θ comprising slurrying a crystalline form of ibandronate sodium characterized by x-ray powder diffraction reflections at 6.2, 25.9, 26.7, 31.1, and 37.2 °2θ ± 0.2 °2θ in 2-butanol.
32. The process of clause 31, wherein the slurry is maintained with stirring for about 0 to about 24 hours to obtain the crystalline form of ibandronate sodium.
33. The process of clause 31 or 32, further comprising heating the slurry.
34. The process of clause 33, wherein the slurry is heated at about 100°C to about 110°C.
35. A pharmaceutical formulation comprising at least one crystalline form of ibandronate sodium selected from the group consisting of:
   a) a crystalline form of ibandronate sodium characterized by x-ray powder diffraction reflections at 5.3, 17.2, 17.8 and 18.4 °2θ ± 0.2 °2θ;
   b) a crystalline form of ibandronate sodium characterized by x-ray powder diffraction reflections at 5.4, 11.4, 16.5 and 17.5 °2θ ± 0.2 °2θ, and
   c) a crystalline form of ibandronate sodium characterized by x-ray powder diffraction reflections at 5.0, 10.9, 13.8 and 17.7 °2θ ± 0.2 °2θ,
   and at least one pharmaceutically acceptable excipient.
36. A process for preparing a pharmaceutical formulation comprising combining at least one crystalline form of ibandronate sodium selected from the group consisting of:
   a) a crystalline form of ibandronate sodium characterized by x-ray powder diffraction reflections at 5.3, 17.2, 17.8 and 18.4 °2θ ± 0.2 °2θ;
   b) a crystalline form of ibandronate sodium characterized by x-ray powder diffraction reflections at 5.4, 11.4, 16.5 and 17.5 °2θ ± 0.2 °2θ, and
   c) a crystalline form of ibandronate sodium characterized by x-ray powder diffraction reflections at 5.0, 10.9, 13.8 and 17.7 °2θ ± 0.2 °2θ, with at least one pharmaceutically acceptable excipient.
37. A method of treating or preventing skeletal-related events comprising administering a pharmaceutical formulation comprising a therapeutically effective amount of at least one crystalline form of ibandronate sodium selected from the group consisting of:
   a) a crystalline form of ibandronate sodium characterized by x-ray powder diffraction reflections at 5.3, 17.2, 17.8 and 18.4 °2θ ± 0.2 °2θ;
   b) a crystalline form of ibandronate sodium characterized by x-ray powder diffraction reflections at 5.4, 11.4, 16.5 and 17.5 °2θ ± 0.2 °2θ, and
   c) a crystalline form of ibandronate sodium characterized by x-ray powder diffraction reflections at 5.0, 10.9, 13.8 and 17.7 °2θ ± 0.2 °2θ,
   and at least one pharmaceutically acceptable excipient to a patient in need thereof.
38. The method of clause 37, wherein the skeletal-related event is osteoporosis.
39. A process for preparing a crystalline form of ibandronate sodium characterized by x-ray powder diffraction reflections at 4.7, 9.2, 17.4, 18.4, and 19.9 °2θ ± 0.2 °2θ comprising combining ibandronate sodium and a solvent to obtain a slurry of the crystalline ibandronate sodium, wherein the solvent is selected from the group consisting of dimethylsulfoxide and ethanol.
40. The process of clause 39, wherein the slurry is stirred for about 20 hours to about 30 hours to obtain the crystalline form of ibandronate sodium.
41. A crystalline form of ibandronate sodium characterized by x-ray powder diffraction reflections at 4.7, 9.2, 17.4, 18.4, and 19.9 °2θ ± 0.2 °2θ, having a maximal particle size of less than about 500 µm.
42. A process for preparing a crystalline form of ibandronate sodium characterized by x-ray powder diffraction reflections at 5.0, 6.1, 17.2, 25.7, and 30.9 °2θ ± 0.2 °2θ comprising storing a crystalline form of ibandronate sodium at a temperature of about 15°C to about 30°C for more than about 3 months,
   wherein the crystalline form of ibandronate sodium that is stored is selected from the group consisting of: a crystalline form of ibandronate sodium characterized by x-ray powder diffraction reflections at 4.9, 6.2, 25.9, 31.0, and 37.1 °2θ ± 0.2 °2θ; a crystalline form of ibandronate sodium characterized by x-ray powder diffraction reflections at 5.3, 6.0, 17.2, 18.7, and 20.0 °2θ ± 0.2 °2θ; and a crystalline form of ibandronate sodium characterized by x-ray powder diffraction reflections at 4.8, 5.1, 5.3, 5.4, and 6.1 °2θ ± 0.2 °2θ.
43. The process of clause 42, wherein the crystalline form of ibandronate sodium is stored for about two years.
44. A process for preparing a crystalline form of ibandronate sodium characterized by x-ray powder diffraction reflections at 6.2, 25.9, 26.7, 31.1, and 37.2 °2θ ± 0.2 °2θ comprising storing a crystalline form of ibandronate sodium at about room temperature for more than about 3 months,
   wherein the crystalline form of ibandronate sodium that is stored is selected from the group consisting of: a crystalline form of ibandronate sodium characterized by x-ray powder diffraction reflections at 4.9, 5.1, 6.0, 20.0, and 36.4 °2θ ± 0.2 °2θ; and a crystalline form of ibandronate sodium characterized by x-ray powder diffraction reflections at 5.0, 10.9, 13.8 and 17.7 °2θ ± 0.2 °2θ.
45. The process of clause 44, wherein the crystalline form of ibandronate sodium is stored for about two years.
46. A process for preparing a crystalline form of ibandronate sodium characterized by x-ray powder diffraction reflections at 6.2, 25.9, 26.7, 31.1, and 37.2 °2θ ± 0.2 °2θ comprising storing a crystalline form of ibandronate sodium at about room temperature for about one week at about 60-100% relative humidity,
   wherein the crystalline form of ibandronate sodium that is stored is selected from the group consisting of: a crystalline form of ibandronate sodium characterized by x-ray powder diffraction reflections at 5.0, 6.1, 17.2, 25.7, and 30.9 °2θ ± 0.2 °2θ; a crystalline form of ibandronate sodium characterized by x-ray powder diffraction reflections at 5.9, 17.1, 19.6, 20.2, and 21.3 °2θ ± 0.2 °2θ; a crystalline form of ibandronate sodium characterized by x-ray powder diffraction reflections at 6.1, 17.2, 19.6, 20.3, and 21.4 °2θ ± 0.2 °2θ; a crystalline form of ibandronate sodium characterized by x-ray powder diffraction reflections at 5.0, 5.9, 17.2, 20.0, and 25.9 °2θ ± 0.2 °2θ; and a crystalline form of ibandronate sodium characterized by x-ray powder diffraction reflections at 5.1, 6.2, 17.3, 19.7, and 20.1 °2θ ± 0.2 °2θ.
47. A process for preparing a crystalline form of ibandronate sodium characterized by x-ray powder diffraction reflections at 6.2, 15.7, 26.3, 32.6, and 35.6 °2θ ± 0.2 °2θ comprising storing amorphous ibandronate sodium at about room temperature for more than about 3 months.
48. The process of clause 47, wherein the amorphous ibandronate sodium is stored for about 2 years.
49. A process for preparing a crystalline form of ibandronate sodium characterized by x-ray powder diffraction reflections at 6.1, 17.2, 19.6, 20.3, and 21.4 °2θ ± 0.2 °2θ comprising storing a crystalline form of ibandronate sodium characterized by x-ray powder diffraction reflections at 5.9, 17.1, 19.6, 20.2, and 21.3 °2θ ± 0.2 °2θ at about room temperature for more than about 3 months.
50. The process of clause 49, wherein the amorphous ibandronate sodium is stored for about 2 years.
51. A process for preparing a crystalline form of ibandronate sodium characterized by x-ray powder diffraction reflections at 4.7, 5.0, 17.2, 18.3 and 19.5 °2θ ± 0.2 °2θ comprising suspending ibandronic acid in n-butanol or 2-butanol; heating the suspension; adding sodium tetraborate decahydrate to the suspension to obtain ibandronate sodium; and cooling the suspension to obtain a precipitate of the crystalline form of ibandronate sodium.
52. The process of clause 51, wherein the suspension is heated to a temperature of about 85°C to about 115°C.
53. The process of clause 51 or 52, wherein the suspension is cooled to a temperature of about 30°C to about 10°C.

## Claims

1. A process for preparing a crystalline form of ibandronate sodium **characterized by** x-ray powder diffraction reflections at 6.2, 15.7, 26.3, 32.6, and 35.6 °2θ ± 0.2 °2θ comprising storing amorphous ibandronate sodium at about room temperature for more than about 3 months.

2. The process of claim 1, wherein the amorphous ibandronate sodium is stored for about 2 years.
